# EUROPÄISCHE PATENTSCHRIFT

(11) **EP 1 273 584 B1**
(45) Veröffentlichungstag und Bekanntmachung des Hinweises auf die Patenterteilung: **18.02.2004**
(21) Anmeldenummer: 02014642.9
(22) Anmeldetag: 02.07.2002
(51) Int. Cl.: C07D 405/06, C07D 491/04

(54) **Verbindungen auf der Basis von 2-bzw. 4-Chromenyliden-Merocyaninen und ihre Verwendung**
2- or 4-Chromenyliden based merocyanines and use thereof
Composés merocyanines basés sur 2- ou 4-chromenyliden et leur utilisation

(30) Priorität: 03.07.2001 DE 10132144; 03.07.2001 DE 10132143
(43) Veröffentlichungstag der Anmeldung: 08.01.2003
(73) Patentinhaber: Dyomics GmbH, 07745 Jena (DE)
(72) Erfinder: Czerney, Peter Dr., 99425 Weimar (DE); Wenzel, Matthias Dr., 07749 Jena (DE); Frank, Wilhelm Dr., 07743 Jena (DE); Lehmann, Frank Dr., 07749 Jena (DE)
(74) Vertreter: Popp, Eugen, Dr.

(56) Entgegenhaltungen:
- GB-A- 2 013 703
- REZENDE M C ET AL: "MEROCYANINE-TYPE DYES FROM BARBITURIC ACID DERIVATIVES" SPECTROCHIMICA ACTA. PART A: MOLECULAR SPECTROSCOPY, PERGAMON PRESS, OXFORD, GB, Bd. 57A, Nr. 6, Mai 2001 (2001-05), Seiten 1183-1190, XP008000820 ISSN: 0584-8539
- DATABASE CROSSFIRE BEILSTEIN [Online] BEILSTEIN INSTITUT ZUR FOERDERUNG DER WISSENSCHAFTEN, FRANKFURT AM MAIN, DE; BRN 5392133, XP002212812 & NTOKOS G ET AL: BULLETIN DE LA SOCIETE CHIMIQUE DE FRANCE, 1976, Seiten 241-243, PARIS, FR
- DATABASE CROSSFIRE BEILSTEIN [Online] BEILSTEIN INSTITUT ZUR FOERDERUNG DER WISSENSCHAFTEN, FRANKFURT AM MAIN, DE; BRN 326914, XP002212813 & WIZINGER ET AL: BERICHTE DER DEUTSCHEN CHEMISCHEN GESELLSCHAFT, Bd. 92, 1959, Seiten 2309-2314, WEINHEIM, DE

## Beschreibung

Die Erfindung betrifft als Fluoreszenzfarbstoffe (Fluorophore) geeignete Verbindungen auf der Basis von Merocyaninen und ihre Verwendung in optischen, insbesondere fluoreszenzoptischen Bestimmungs- und Nachweisverfahren. Typische Verfahrensanwendungen beruhen auf der Reaktion von farbstoffmarkierten Biomolekülen, wie zum Beispiel Antigenen, Antikörpern oder DNA-Segmenten mit der jeweils komplementären Spezies. Damit werden unter anderem Messungen von Enzymkinetiken, Rezeptor-Ligand-Interaktionen und Nucleinsäure-Hybridisierungskinetiken ermöglicht. Des weiteren sind die beanspruchten Fluorophore für die pharmakologische Charakterisierung von Rezeptoren oder Wirkstoffen interessant.

Einsatzmöglichkeiten ergeben sich dadurch beispielsweise in der Medizin und Pharmazie, in der Bio- und Materialwissenschaft, bei der Umweltkontrolle und dem Nachweis von in der Natur und Technik vorkommenden organischen und anorganischen Mikroproben sowie anderes mehr.

Während Cyanine als Fluoreszenzmarker seit langem bekannt sind (US-PS 5 627 027), finden Merocyanin-Verbindungen bisher nur vereinzelt Anwendung. Typische Beispiele hierfür sind die Marker vom Typ *AMCA* (7-Amino-4-methylkumarin-3-essigsäure; US-PS 5 696 157 und US-PS 4 956 480). Das Charakteristische an diesen Verbindungen ist, dass deren Absorptionsbanden im UV- bzw. am Anfang des sichtbaren Spektralbereichs liegen. Entsprechend kann die Emission nur im Bereich unter 500 nm beobachtet werden.

Merocyanine, z. B. das *Merocyanin 540*, werden auch zum Nachweis und zur Quantifizierung von Peptiden, Polypeptiden und Proteinen verwendet (US-PS 5 616 502). Der Nachteil der in der US-PS 5 616 502 beanspruchten Fluoreszenzmarker besteht darin, dass sie keine Möglichkeit zur Einführung einer reaktiven Funktion, die eine kovalente Kopplung mit einem nucleophilen Biomolekül ermöglicht, besitzen.

Der Erfindung liegt die Aufgabe zu Grunde, als Fluoreszenzmarker geeignete Verbindungen auf der Basis von Merocyaninen mit hoher Photo- und Lagerstabilität sowie hoher Fluoreszenzquantenausbeute zu schaffen. Um ein optimales Signal/Rausch-Verhältnis zu erreichen, sollten die Emissionsbanden im Bereich über 550 nm liegen und die Anregung auf möglichst einfache Weise durch Weißlicht- oder Laser-Strahlung im UV-, sichtbaren oder nahen infraroten Spektralbereich realisiert werden. Die Fluorophore sollten möglichst eine vom pH-Wert und anderen Umwelteinflüssen unabhängig hohe Fluoreszenz aufweisen. Voraussetzung für die kovalente Anbindung ist das Vorhandensein einer reaktiven Funktion, die unter physiologischen Bedingungen bzw. unter den bei der Festphasensynthese von Biooligomeren üblichen Reaktionsbedingungen mit dem zu markierenden Biomolekül reagiert.

Gegenstand der Erfindung sind nun neue Verbindungen auf der Basis von Merocyaninen der allgemeinen Formeln **I** und **II,** 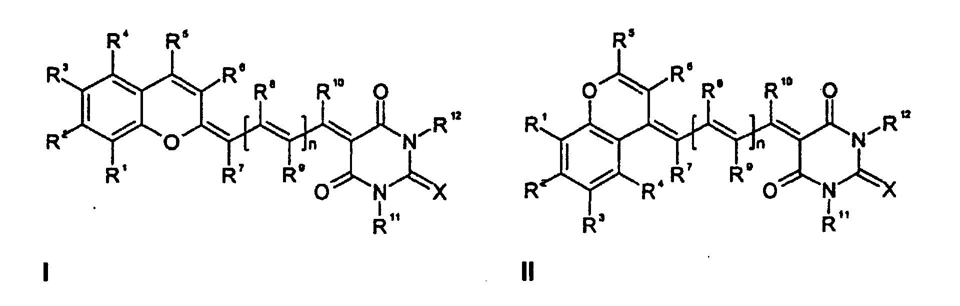
- wobei R¹ - R¹² gleich oder unterschiedlich sind und Wasserstoff, ein oder mehrere Chlor- und/oder Bromatome, Alkyl-, Aryl-, Heteroaryl-, Cycloalkyl-, Heterocycloalkyl-, Alkyloxy-, Alkylmercapto- (wobei die Begriffe Alkyl und Cycloalkyl auch Reste mit olefinischen Bindungen einschließen sollen), Aryloxy-, Arylmercapto-, Heteroaryloxy-, Heteroarylmercapto- oder Cyano-Reste, eine oder mehrere Hydroxy-, eine oder mehrere alkylsubstituierte oder cyclische Aminofunktionen sein können und R¹ und R², R² und R³, R³ und R⁴; R⁵ und R⁶ und/bzw. R⁶ und R⁷ einen oder mehrere aliphatische, heteroaliphatische oder aromatische Ringe bilden können,
- X für ein Element aus der Gruppe O, S, Se, Te oder das Strukturelement CR₂ oder NR, wobei R die Funktionen von R¹ - R¹² einnehmen kann, steht,
- einer oder mehrere der Substituenten R¹ - R¹² solubilisierende bzw. ionisierbare bzw. ionisierte Substituenten wie SO₃⁻, PO₃²⁻, CO₂H, OH, NR₃⁺, Cyclodextrin oder Zucker darstellen können, die die hydrophilen Eigenschaften der Farbstoffe bestimmen, wobei diese Substituenten auch über eine aliphatische oder heteroaliphatische, gegebenenfalls zyklische Spacergruppe am eigentlichen Grundchromophor angebunden sein können,
- mindestens einer der Substituenten R¹ - R¹² für eine reaktive Gruppe vom Typ Isocyanat, Isothiocyanat, Hydrazin, Amin, Mono- und Dichlor- bzw. Mono- und Dibromtriazin, Aziridin, Sulfonylhalogenid, *N*-Hydroxysuccinimidester, Imido-Ester, Glyoxal oder Aldehyd bzw. Maleimid oder lodacetamid sowie Phosphoramidit steht, wobei der jeweilige Substituent über eine aliphatische oder heteroaliphatische, gegebenenfalls zyklische Spacergruppe am eigentlichen Grundchromophor angebunden sein kann,
- die aliphatische oder heteroaliphatische Spacergruppe aus einem Strukturelement -[(CH₂)ₐ-Y-(CH₂)_{b}]_{c}- besteht, worin Y gleich oder verschieden eine CR₂-, O-, S-, SO₂, SO₂NH-, NR-, COO- oder CONR- Funktion sein kann, wobei R die Funktionen von R¹ - R¹² einnehmen kann und a und b gleich oder verschieden die Werte von 0 - 18 und c die Werte von 1-18 darstellen,
- n für die Zahlenwerte 0, 1, 2 oder 3 steht, wobei die für n = 2 oder 3 jeweilig doppelt oder dreifach vorkommenden Substituenten R⁸ und R⁹ gleich oder unterschiedlich sein können,
- CR¹⁰ für eine der Struktureinheiten 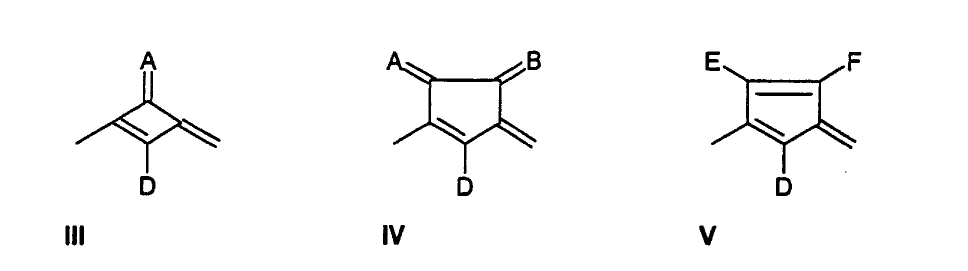 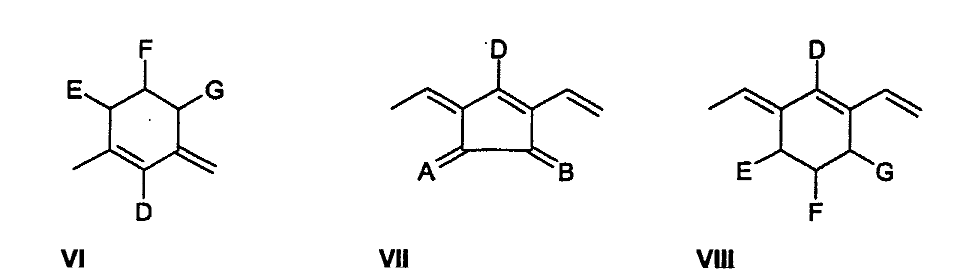 stehen,
- die Substituenten A und B gleich oder unterschiedlich jeweils für zwei Substituenten vom Typ R¹ - R¹², für CR₂ oder N-R, wobei R gleich oder unterschiedlich die Funktionen von R¹ - R¹² einnehmen kann, bzw. für O oder S stehen und die Substituenten D - G gleich oder unterschiedlich die Funktionen von R¹ - R¹² einnehmen können.

Typische erfindungsgemäße Verbindungen sind insbesondere solche der Formeln **I** und **II**, in denen R¹ bis R¹² gleich oder unterschiedlich sind und Wasserstoff, Chlor, Brom, einen aliphatischen oder einkemigen aromatischen Rest, jeweils mit insgesamt höchstens 12, vorzugsweise höchstens 10, insbesondere höchstens 7 Kohlenstoffatomen bedeuten, die als substituierter Rest außer Kohlenstoff und Wasserstoff noch bis zu 4 Sauerstoffatome und entweder 0, 1 oder 2 Stickstoffatome oder ein Schwefelatom oder ein Schwefel- und ein Stickstoffatom enthalten können oder eine Aminfunktion sind, an deren Stickstoffatom Wasserstoff oder Substituenten mit zusammen höchstens 8 Kohlenstoffatomen gebunden sind, wobei diese Substituenten aus Kohlenstoff, Wasserstoff und höchstens zwei Sulfonsäuregruppen bestehen, wobei R¹ und R², R² und R³, R³ und R⁴, R⁵ und R⁶ und R⁶ und R⁷ jeweils zusammen einen aromatischen oder nichtaromatischen Kohlenwasserstoff- oder stickstoffhaltigen 5- oder 6-Ring bilden können, mit der Maßgabe, dass R² eine Aminfunktion darstellt und dass wenigstens einer der Reste R¹ und R³ für sich einen SO₃⁻ -Rest darstellen kann,
X Sauerstoff oder Schwefel bedeutet, wobei Sauerstoff bevorzugt ist, und
n = 0 oder eine ganze Zahl von 1 - 3 bedeutet, wobei die für n = 2 oder 3 doppelt oder dreifach vorkommenden Substituenten R⁸ und R⁹ gleich oder verschieden sein können, jedoch n bevorzugt 0 oder 1 ist.

Eine spezielle Gruppe der erfindungsgemäßen Verbindungen sind solche, in denen R², R¹¹ und R¹² frei von Chlor und Brom sind, in denen aber sonst in höchstens 4, zweckmäßig in höchstens 2 der Reste R¹ und R³ bis R¹⁰ Chlor und/oder Brom enthalten ist. Bevorzugt ist dass alle Reste R¹ und R³ - R¹⁰ frei von Chlor und Brom sind.

Einige bevorzugte Verbindungen enthalten aliphatische Reste R¹ - R¹² mit 2 - 6 Kohlenstoffatomen, die in ω-Stellung durch eine reaktive Gruppe, z.B. SO₃⁻-, SO₂Cl-, HO-, oder Carboxylgruppe substituiert sind, die ihrerseits zu Estern umgesetzt werden können, z.B. mit N-Hydroxysuccinimid, Maleimid oder Phosphoramidit, oder auch mit den oben genannten reaktiven Gruppen vom Typ Isocyanat und dergleichen. Derartige Gruppen bewirken, dass die erfindungsgemäßen Verbindungen zur Reaktion mit Biomolekülen unter Ausbildung einer kovalenten Bindung fähig sind.

Geeignete Verbindungen sind insbesondere auch solche, in denen die Reste R¹ bis R¹² außer der Aminfunktion von R² insgesamt höchstens 2 Stickstoffatome enthalten.

Soweit Reste R¹ bis R¹² Kohlenwasserstoffreste sind, sind solche mit 1 - 4 Kohlenstoffatomen bevorzugt.

In einer Gruppe von bevorzugten Verbindungen enthält wenigstens einer der Reste R¹ bis R¹² eine solubilisierende oder ionisierbare Gruppe, die vorzugsweise über eine aliphatische oder heteroaliphatische Gruppe an das Grundgerüst gebunden ist. Der Begriff ionisierbar wird so verstanden, dass er auch ionisiert umfasst. Derartige solubilisierende bzw. ionisierbare Gruppen sind insbesondere SO₃⁻, CO₂H oder OH, des weiteren auch PO₃²⁻, NR₃⁺, Cyclodextrin oder Zucker. Derartige Gruppen verleihen den erfindungsgemäßen Verbindungen zusätzliche hydrophile Eigenschaften, die insbesondere die Fluoreszenzquantenausbeute positiv beeinflussen.

Die erfindungsgemäßen Verbindungen können als Farbstoffe zur optischen Markierung von Proteinen, Nukleinsäuren, Oligomeren, DNA, RNA, biologischen Zellen, Lipiden, Mono-, Oligo- und Polysacchariden, Liganden, Rezeptoren, Polymeren, Pharmaka oder Polymerpartikeln verwendet werden und als Farbstoffe in Systemen zur qualitativen oder quantitativen Bestimmung von Proteinen, Nukleinsäuren, Oligomeren, DNA, RNA, biologischen Zellen, Lipiden, Polymeren, Pharmaka oder Polymerpartikeln über die funktionellen Gruppen an eine HO-, H₂N-, HS-oder HO₂C-Funktion der zu bestimmenden Substanzen gekoppelt werden.

Diese Kopplungsreaktion wird vorteilhaft in organischen oder wäßrigen Lösungen durchgeführt.

Die aus den erfindungsgemäßen Verbindungen und Biomolekülen bestehenden Konjugate weisen fluoreszierende Eigenschaften auf.

Die erfindungsgemäßen Verbindungen finden in optischen, insbesondere fluoreszenzoptischen qualitativen und quantitativen Bestimmungsverfahren einschließlich Immuntests, Hybridisierungsverfahren, chromatographischen oder elektrophoretischen Verfahren und Hoch-Durchsatz-Screenings oder zur Analyse von Rezeptor-Liganden-Wechselwirkungen auf einem Mikroarray Verwendung.

Die Merocyanine der allgemeinen Formeln I und/oder 11 können als Farbstoffe zur optischen Markierung von organischen oder anorganischen Erkennungseinheiten, z. B. von Aminosäuren, Peptiden, Proteinen, Antigenen, Haptenen, Enzymsubstraten, Enzym-Cofaktoren, Biotin, Carotinoiden, Hormonen, Neurohormonen, Neurotransmittern, Wachstumsfaktoren, Lympholocinen, Lektinen, Toxinen, Kohlenhydraten, Oligosacchariden, Polysacchariden, Dextranen, Nucleinsäuren, Oligonucleotiden, DNA, RNA, biologischen Zellen, Lipiden, rezeptorbindenden Pharmaka oder organischen bzw. anorganischen polymeren Trägermaterialien verwendet werden.

Die Markierung der Erkennungseinheiten kann dabei durch die Ausbildung von ionischen Wechselwirkungen zwischen den Verbindungen der allgemeinen Formeln **I** und/oder **II** und den zu markierenden Materialien erfolgen.

Weiterhin besteht auch die Möglichkeit, die Erkennungseinheit oder das Trägermaterial kovalent mit dem Fluorophor zu verbinden. Diese Kopplungsreaktion kann in wäßriger oder überwiegend wäßriger Lösung und vorzugsweise bei Raumtemperatur durchgeführt werden. Dabei entsteht eine Fluoreszenz-Sonde (Konjugat) zur qualitativen oder quantitativen Bestimmung von unterschiedlichen Biomaterialien bzw. anderen organischen und anorganischen Materialien.

Sowohl die Verbindungen der allgemeinen Formeln **I** und/oder **II** und davon abgeleitete Systeme können in optischen, insbesondere fluoreszenzoptischen, qualitativen und quantitativen Bestimmungsverfahren zur Diagnostik von Zelleigenschaften, in Biosensoren (*point of care*-Messungen), zur Erforschung des Genoms und in Miniaturisierungstechnologien eingesetzt werden. Typische Anwendungen erfolgen in der Zytometrie und Zellsortierung, der Fluoreszenz-Korrelations-Spektroskopie (FCS), im *Ultra-High-Throughput-Screening* (UHTS), bei der *multicolor* Fluoreszenz*-in-situ*-Hybridisierung (FISH) und in Mikroarrays (DNAund Protein-Chips).

Dabei ist ein Mikroarray eine rasterartige Anordnung von auf mindestens einer Oberfläche immobilisierten Molekülen, die zum Studium von Rezeptor-Liganden-Wechselwirkungen verwendet werden können. Eine rasterartige Anordnung bedeutet mehr als zwei voneinander verschiedene Moleküle, welche sich innerhalb einer Fläche befinden und dort in unterschiedlichen, vorher definierten Regionen mit bekannter Position immobilisiert sind.

Ein Rezeptor ist ein Molekül, das eine Affinität zu einem gegebenen Liganden besitzt. Rezeptoren können natürlich vorkommende oder künstlich hergestellte Moleküle sein. Rezeptoren können in reiner Form oder gebunden an andere Spezies eingesetzt werden. Rezeptoren können kovalent oder nichtkovalent entweder direkt oder durch bestimmte Kopplungsvermittler an einen Bindungspartner angeknüpft werden.

Beispiele für Rezeptoren, die durch diese Erfindung detektiert werden können, schließen Agonisten und Antagonisten für Zell-Membran-Rezeptoren, Toxine und andere Giftstoffe, virale Epitope, Hormone wie Opiate und Steroide, Hormonrezeptoren, Peptide, Enzyme, Enzymsubstrate, als Kofaktoren agierende Wirkstoffe, Lektine, Zucker, Oligonukleotide, Nukleinsäuren, Oligosaccharide, Zellen, Zellfragmente, Gewebefragmente, Proteine und Antikörper ein, sind aber nicht auf die angeführten Stoffe beschränkt.

Ein Ligand ist ein Molekül, das von einem bestimmten Rezeptor erkannt wird. Beispiele für Liganden, die durch die erfindungsgemäßen Verbindungen detektiert werden können, schließen Agonisten und Antagonisten für Zell-Membran-Rezeptoren, Toxine und andere Giftstoffe, virale Epitope, Hormone wie Opiate und Steroide, Hormonrezeptoren, Peptide, Enzyme, Enzymsubstrate, als Kofaktoren agierende Wirkstoffe, Lektine, Zucker, Oligonukleotide, Nukleinsäuren, Oligosaccharide, Proteine und Antikörper ein, sind aber nicht auf die angeführten Stoffe beschränkt.

Durch die Darstellung von nichtsymmetrischen Merocyaninen, die einerseits als terminale Funktion einen leicht derivatisierbaren Heterocyclus vom Typ CH-acider Verbindungen, andererseits einen neuartig substituierten 6-Ringheterocyclus aufweisen, werden insbesondere nachfolgende Vorteile erreicht:

Bereits relativ kleine Moleküle absorbieren im Spektralbereich über 550 nm und zeigen gegenüber den bisher bekannten Polymethinen mit Absorptionsmaxima über 650 nm (Penta- und Heptamethine) eine wesentlich verbesserte photochemische und thermische Stabilität.

Durch *molecular engineering* ist es möglich, Lage und Intensität der Absorptions- und Emissionsmaxima beliebig zu steuern und den Emissionswellenlängen unterschiedlicher Anregungslaser, vor allem Diodenlasern, anzupassen.

Bedingt durch die Auswahl geeigneter terminaler Heterocyclen zeigen die erfindungsgemäßen Farbstoffe zusätzliche Absorptionsmaxima im sichtbaren bzw. NIR-Spektralbereich, die zur Anregung, beispielsweise mit einem Argon-Laser, genutzt werden können. Diese Farbstoffe sind insbesondere zur Anwendung in *multiple color fluorescence assays* geeignet.

Die erfindungsgemäßen Verbindungen sind relativ einfach durch das Kondensieren der beiden unterschiedlichen CH-aciden Heterocyclen und eines C-1, C-3 oder C-5 Baustein herzustellen (Eintopf-Verfahren).

Weitere Verfahren zu ihrer Herstellung bestehen darin, daß in einer 1. Reaktionsstufe einer der CH-aciden Heterocyclen mit dem C-1, C-3 oder C-5 Baustein kondensiert wird und nach Isolierung des 1:1-Kondensationsproduktes in einer nachfolgenden Kondensation mit dem zweiten CH-aciden Heterocyclus zum Merocyanin umgesetzt wird. Dabei ist die Reihenfolge der Verwendung der Heterocyclen unerheblich. Dadurch ist in wenigen Reaktionsschritten eine Vielzahl unterschiedlich funktionalisierter Farbstoffe, die sich hinsichtlich der Gesamtladung und der Spezifität/Reaktivität der zur Immobilisierung genutzten aktivierten Gruppen unterscheiden, auf einfache Weise herstellbar.

Gegenstand der Erfindung ist weiterhin die Verwendung der erfindungsgemäßen Verbindungen als Farbstoffkomponente zur optischen Markierung biologischer und organischer Substanzen, wie Proteinen, Nukleinsäuren und den anderen oben genannten Stoffen.

Die erfindungsgemäßen Verbindungen eignen sich als Fluoreszenzfarbstoffe (Fluorophore) zur Verwendung in optischen, insbesondere fluoreszenz-optischen Bestimmungs- und Nachweisverfahren, beispielsweise in der Medizin, in der Pharmazie sowie in der Bio-, Material- und Umweltwissenschaft. Sie sind durch eine hohe Photo- und Lagerstabilität gekennzeichnet und weisen eine hohe Fluoreszenzquantenausbeute auf. Sie können auf einfache Weise durch Weißlichtquellen oder durch Laserstrahlung im UV-, sichtbaren oder NIR-Spektralbereich angeregt werden.

Die Erfindung soll nachstehend anhand von in der Zeichnung dargestellten Ausführungsbeispielen näher erläutert werden.

Es zeigen:
- Fig. 1:: Synthese vom Natriumsalz des *N*-(5-Ethoxycarbonyl)-pentyl-*N'*-3-sulfonatopropyl-harnstoffs, Vorprodukt **A**
- Fig. 2:: Synthese vom Natriumsalz des 1-*N*-(5'-Ethoxycarbonyl)-pentyl-3-*N'*-3'sulfonatopropyl-2,4,6-trioxo-5*H*-pyrimidins, Vorprodukt **B**
- Fig. 3:: Synthese vom Natriumsalz des 3-[5-{2-[4-(*tert*-Butyl)-7-(diethylamino)-2*H*-2-chromenyliden]ethyliden}-3-(5-carboxypentyl)-2,4,6-trioxohexahydro-1-pyrimidinyl]-1-propansulfonats (**F 39/DY-610**), Ausführungsbeispiel **1**
- Fig. 4:: Synthese des *N*-Hydroxysuccinimid-Esters (NHS) von **F 39,** Ausführungsbeispiel **2**
- Fig. 5:: Synthese vom Natriumsalz des 3-[5-{[9-(*tert*-Butyl)-6-(diethylamino)-1,2-dihydrocyclopenta[*b*]chromen-3-yl]methylen}-3-(5-carboxypentyl)-2,4,6-trioxohexahydro-1-pyrimidinyl]-1-propansulfonats (**F 46**), Ausführungsbeispiel **3**
- Fig. 6:: Synthese vom Natriumsalz des 3-[5-{2-[4-(Phenyl)-7-(diethylamino)-2*H*-2-chromenyliden]ethyliden}-3-(5-carboxypentyl)-2,4,6-trioxohexahydro-1-pyrimidinyl]-1-propansulfonats (**F 40**), Ausführungsbeispiel **4**
- Fig. 7:: Synthese vom Natriumsalz des 3-[5-{2-[3-(Methyl)-4-(phenyl)-7-(diethylamino)-2*H*-2-chromenyliden]ethyliden}-3-(5-carboxypentyl)-2,4,6-trioxohexahydro-1-pyrimidinyl]-1-propansulfonats (**F 41**), Ausführungsbeispiel **5**
- Fig. 8:: Synthese des NHS-Esters von **F 41,**
Ausführungsbeispiel **6**
- Fig. 9:: Synthese von 1,3-Dibutyl-5-{[9-(2-carboxyphenyl)-6-(diethylamino)-2,3-dihydro-1*H*-4-xanthenyl]methylen}-hexahydro-2,4,6-pyrimidintrion (**F 33**),
Ausführungsbeispiel **7**
- Fig. 10:: Synthese von *N*-(3-Hydroxypropyl)-*N*'-butylharnstoff, Vorprodukt **C**
- Fig. 11:: Synthese von 1-*N*-(3'-Hydroxypropyl)-3-*N'*-butyl-2,4,6-trioxo-5*H*pyrimidin, Vorprodukt **D**
- Fig. 12:: Synthese von 1-Butyl-5-{2-[3-(methyl)-4-(phenyl)-7-(diethylamino)-2*H*-2-chromenyliden]ethyliden}-3-(3-hydroxypropyl)hexahydro-2,4,6-pyrimidintrion (**F 45**),
Ausführungsbeispiel **8**
- Fig. 13:: Synthese des Phosphoramidits von **F 45**,
Ausführungsbeispiel **9**
- Fig. 14:: Synthese von 1,3-Dibutyl-5-{2-[2-(*tert*-butyl)-7-(diethylamino)-4*H*-4-chromenyliden]ethyliden}-hexahydro-2,4,6-pyrimidintrion (**F 34**),
Ausführungsbeispiel **10**
- Fig. 15:: Synthese vom Natriumsalz des 3-[5-{2-[2-(*tert*-Butyl)-7-(diethylamino)-4*H*-4-chromenyliden]ethyliden}-3-(5-carboxypentyl)-2,4,6-trioxohexahydro-1-pyrimidinyl]-1-propansulfonats (**F 37/DY-550**),
Ausführungsbeispiel **11**
- Fig. 16:: Synthese des NHS-Esters von **F 37**,
Ausführungsbeispiel **12**
- Fig. 17:: Synthese vom Dinatriumsalz des 3-[5-(2-{2-*tert*-Butyl-7-[ethyl-(3-sulfonato-propyl)-amino]-4*H*-4-chromenyliden}-ethyliden)-3-(5-carboxypentyl)-2,4,6-trioxohexahydro-1-pyrimidinyl]-1-propansulfonats (**V03-01173**),
Ausführungsbeispiel **13**
- Fig. 18:: Synthese vom Natriumsalz des 3-[5-{(E)-4-[2-(*tert*-Butyl)-7-(diethylamino)-4*H*-4-chromenyliden]-2-butenyliden}-3-(5-carboxypentyl)-2,4,6-trioxohexahydro-1-pyrimidinyl]-1-propansulfonats (**F 38**),
Ausführungsbeispiel **14**
- Fig. 19:: Synthese vom Natriumsalz des 3-[5-{2-[2-(*tert*-Butyl)-9-ethyl-6,8,8-trimethyl-8,9-dihydro-4*H*-pyrano-[3,2-*g*]chinolin-4-yliden]ethyliden}-3-(5-carboxy-pentyl)-2,4,6-trioxohexahydro-1-pyrimidinyl]-1-propansulfonats (**F 48**), Ausführungsbeispiel **15**
- Fig. 20:: Synthese vom Natriumsalz des 3-[5-{4-[2-(*tert*-Butyl)-9-ethyl-6,8,8-trimethyl-8,9-dihydro-4*H*-pyrano-[3,2-*g*]chinolin-4-yliden]-2-butenyliden}-3-(5-carb-oxypentyl)-2,4,6-trioxohexahydro-1-pyrimidinyl]-1-propansulfonats (**F 50**), Ausführungsbeispiel **16**
- Fig. 21:: Synthese des NHS-Esters von **F 50**,
Ausführungsbeispiel **17**
- Fig. 22:: Absorptions- und Emissionsspektrum eines DY-610-DNA-Konjugats, hergestellt durch NHS-Kopplung an das 5'-aminomodifizierte Oligomer mit anschließender Hochdruckflüssigkeitschromatographie-(HPLC-) Reinigung, gemessen in destilliertem Wasser, Ausführungsbeispiel **18**
- Fig. 23:: Absorptions- und Emissionsspektrum eines DY-550-Avidin-Konjugats, hergestellt über NHS-Ester-Kopplung in Bicarbonat-Puffer bei pH 9,0 mit anschließender Aufreinigung über Sephadex G25 medium (Eluent Phosphatpuffer, 22 mM, pH 7,2),
Ausführungsbeispiel **19**

Im folgenden bezieht sich der Begriff "Vakuum" auf den Druckbereich von 30 ― 150 mbar. Die Mischungsverhältnisse von Flüssigkeiten sind Volumenverhältnisse. RT bedeutet Raumtemperatur.

### Herstellung der Vorprodukte A und B

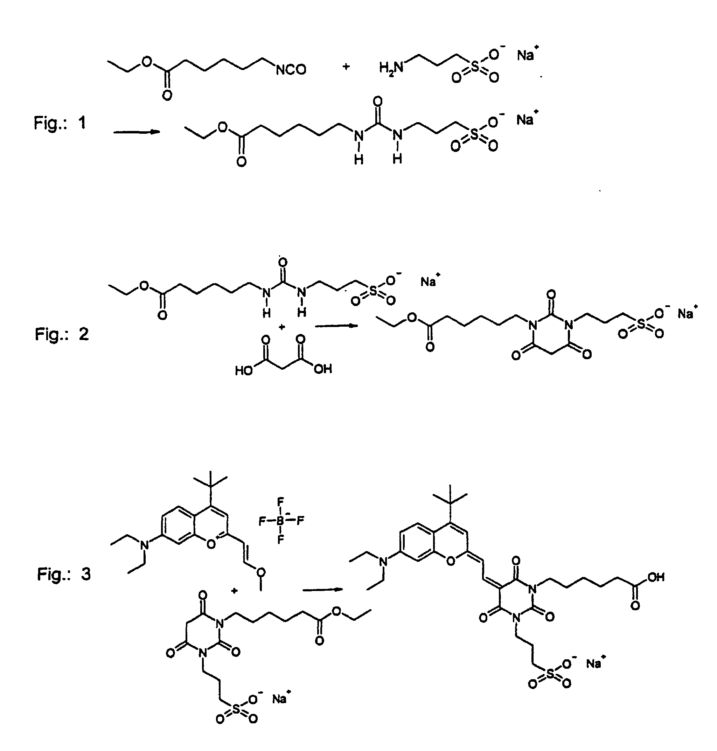

### A. Natriumsalz des N-(5-Ethoxycarbonyl)-pentyl-N'-3-sulfonatopropyl-harnstoffs, vgl. Fig. 1:

696 mg (5,0 mmol) 3-Aminopropansulfonsäure werden in 2 ml Wasser unter Zusatz von 200 mg (5,0 mmol) NaOH gelöst. Nach dem Verdünnen mit 1 ml Ethanol gibt man unter Rühren portionsweise 1315 mg (7,1 mmol) 6-Isocyanatohexansäureethylester zu, wobei die Temperatur auf etwa 40 °C ansteigt. Im Anschluß daran wird die Temperatur für 10 Minuten auf 70 °C gehalten. Nach dem Abkühlen wird das Lösungsmittelgemisch im Vakuum abgezogen und der farblose Rückstand mit Aceton ausgekocht. Der in Aceton unlösliche Rückstand wird abfiltriert, mit warmem Aceton gewaschen und getrocknet. 1,52 g (88 %) Ausbeute. ― ¹H NMR (250 MHz, D₂O): 1.21 (t, 3H), 1.29 (m, 2H), 1.43 (m, 2H), 1.58 (m, 2H), 1.86 (m, 2H), 2.34 (t, 2H), 2.88 (m, 2H), 3.05 (t, 2H), 3.18 (t, 2H), 4.12 (q, 2H). ― MS (ESI^{⊖}): 323 (M^{⊖}). ― C₁₂H₂₃NaN₂O₆S (346,37).

### B. Natriumsalz des 1-N-(5'-Ethoxycarbonyl)-pentyl-3-N'-3'-sulfonatopropyl-2,4,6-trioxo-5H-pyrimidins, vgl. Fig. 2:

1473 mg (4,25 mmol) Natriumsalz des *N*-(5-Ethoxycarbonyl)-pentyl-*N'*-3-sulfonatopropyl-harnstoffs und 468 mg (4,5 mmol) Malonsäure werden in 4 ml Essigsäure gelöst und auf 60 °C erwärmt. Zu dieser Lösung tropft man langsam 8 ml Acetanhydrid und hält die Lösung für 6 Stunden auf 100 °C. Nach dem Abkühlen auf Raumtemperatur wird das Lösungsmittel im Vakuum abgezogen und der Rückstand mit Ethanol versetzt. Der ausfallende Niederschlag wird abgesaugt und durch Umkristallisation aus Ethanol gereinigt. 1,32 g (75 %) Ausbeute. ― ¹H NMR (250 MHz, D₂O): 1.13 (t, 3H), 1.22 (m, 2H), 1.53 (m, 4H), 1.92 (m, 2H), 2.26 (m, 2H), 2.54 (s, 2H), 2.83 (t, 2H), 3.71 (m, 2H), 3.85 (m, 2H), 4.03 (q, 2H). ― MS (ESI^{⊖}): 391 (M^{⊖}). ― C₁₅H₂₃NaN₂O₈S (414,40).

### Ausführungsbeispiele 1 ― 7 (Verbindungen nach der allgemeine Formel I)

### 1. Natriumsalz des 3-[5-{2-[4-(tert-Butyl)-7-(diethylamino)-2H-2-chromenyliden]ethyliden}-3-(5-carboxypentyl)-2,4,6-trioxohexahydro-1-pyrimidinyl]-1-propansulfonats (F 39/DY-610), vgl. Fig. 3:

### Variante A

241 mg (0,60 mmol) 4-(*tert*-Butyl)-7-(diethylamino)-2-[(*E*)-2-methoxy-1-ethenyl]-chrome-nium-tetrafluoroborat und 249 mg (0,60 mmol) Natriumsalz des 1-*N*-(5'-Ethoxycarbonyl)-pentyl-3-*N*'-3'-sulfonatopropyl-2,4,6-trioxo-5H-pyrimidins werden in 20 ml Ethanol gelöst, mit ca. 1 ml Triethylamin versetzt und kurz auf etwa 70 °C erwärmt. Nach dem Abkühlen auf Raumtemperatur beläßt man noch für 30 Minuten, zieht dann das Lösungsmittel im Vakuum ab und chromatographiert den Rückstand (SiO₂, Eluent Toluol/Ethanol im Verhältnis 7:3).
Der isolierte Ester wird in einer Mischung aus 10 ml Aceton und 10 ml 2-molarer Salzsäure 2 Stunden unter Rückfluß gekocht, die abgekühlte Reaktionslösung mit NaHCO₃ neutralisiert und das Lösungsmittel im Vakuum abgezogen. Der in Methanol lösliche Rückstand wird erneut chromatographiert (SiO₂ RP 18, Eluent Methanol/Wasser im Verhältnis 6:4).
170 mg (42 %) Ausbeute. ― UV/Vis (Ethanol): λₘₐₓ (ε) = 609 nm (190.000 |· mol⁻¹· cm⁻¹). ― Fluoreszenz (Ethanol): λₑₘ = 629 nm. ― ¹H NMR (250 MHz, D₂O): 1.00 (t, 6H), 1.25 (m, 2H), 1.27 (s, 9H), 1.43 (m , 4H), 1.87 (m 2H), 2.08 (t, 2H), 2.79 (t, 2H), 3.20 (s, br, 4H), 3.55 (s, br, 2H), 3.71 (s, br, 2H), 6.26 (m, 2H), 6.45 (d, 1H), 6.58 (d, 1 H), 7.70 (d, 1 H), 7.79 (d, 1H). ― MS (ESI^{⊖}): 644 (M^{⊖}), 322 (M-H)^{2⊖}. ― C₃₂H₄₂NaN₃O₉S (667,74).

### Variante B

216 mg (0,60 mmol) 4-(*tert*-Butyl)-7-(diethylamino)-2-methylchromeniumtetrafluoroborat und 274 mg (0,60 mmol) Natriumsalz des 1-*N*-(5'-Ethoxycarbonyl)-pentyl-5-(methoxy-methylen)-3-*N'*-3'-sulfonatopropyl-2,4,6-trioxohexahydro-1-pyrimidins werden in 20 ml Ethanol gelöst, mit ca. 1 ml Triethylamin versetzt und kurz auf etwa 70 °C erwärmt. Die weitere Aufarbeitung der Reaktionslösung erfolgt nach Variante A.

### Variante C

216 mg (0,60 mmol) 4-(*tert*-Butyl)-7-(diethylamino)-2-methylchromeniumtetrafluoroborat, 249 mg (0,60 mmol) Natriumsalz des 1-*N*-(5'-Ethoxycarbonyl)-pentyl-3-*N'*-3'-sulfonato-propyl-2,4,6-trioxo-5H-pyrimidins, 1 ml Trimethoxymethan und 1 ml Pyridin werden in 10 ml Acetanhydrid gelöst und 1 Stunde unter Rückfluß erhitzt. Die weitere Aufarbeitung der Reaktionslösung erfolgt nach Variante A.

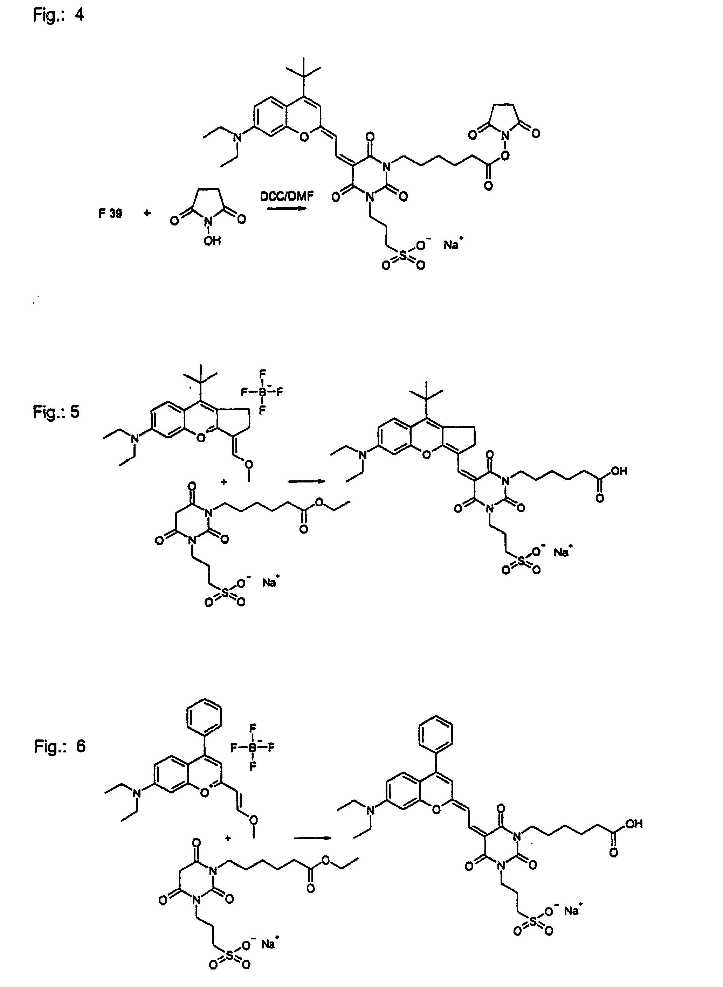

### 2. Darstellung des NHS-Esters von F 39 mit N-Hydroxysuccinimid (NHS)/N,N'-Dicyclo-hexylcarbodiimid (DCC), vgl. Fig. 4:

15 mg **F 39,** 14 mg DCC und 4 mg NHS werden in 1 ml trockenem DMF gelöst. Dann gibt man 10 µl Triethylamin zu. Das Reaktionsgemisch wird 24 Stunden bei Raumtemperatur gerührt und anschließend filtriert. Im Anschluß daran wird das Lösungsmittel abgezogen und der Rückstand mit Diethylether gewaschen. Diese Reaktion verläuft quantitativ.

### 3. Natriumsalz des 3-[5-{[9-(tert-Butyl)-6-(diethylamino)-1,2-dihydrocyclopenta[b]chromen-3-yl]methylen}-3-(5-carboxypentyl)-2,4,6-trioxohexahydro-1-pyrimidinyl]-1-propansulfonats (F 46), vgl. Fig. 5:

256 mg (0,60 mmol) 9-(*tert*-Butyl)-6-(diethylamino)-3-[(*E*)-1-methoxymethyliden]-1*H*,2*H*, 3*H*-cyclopenta[*b*]chromenium-tetrafluoroborat und 249 mg (0,60 mmol) Natriumsalz des 1-*N*-(5'-Ethoxycarbonyl)-pentyl-3-*N'*-3'-sulfonatopropyl-2,4,6-trioxo-5H-pyrimidins werden nach Ausführungsbeispiel 1, Variante A zur Reaktion gebracht und aufgearbeitet.
158 mg (38 %) Ausbeute. - UV/Vis (Ethanol): λₘₐₓ (ε) = 644 nm (45.700 |· mol⁻¹· cm⁻¹). ― Fluoreszenz (Ethanol): λₑₘ = 670 nm. ― MS (ESI^{⊖}): 670 (M^{⊖}), 335 (M-H)^{2⊖}. ― C₃₄H₄₄NaN₃O₉S (693,78).

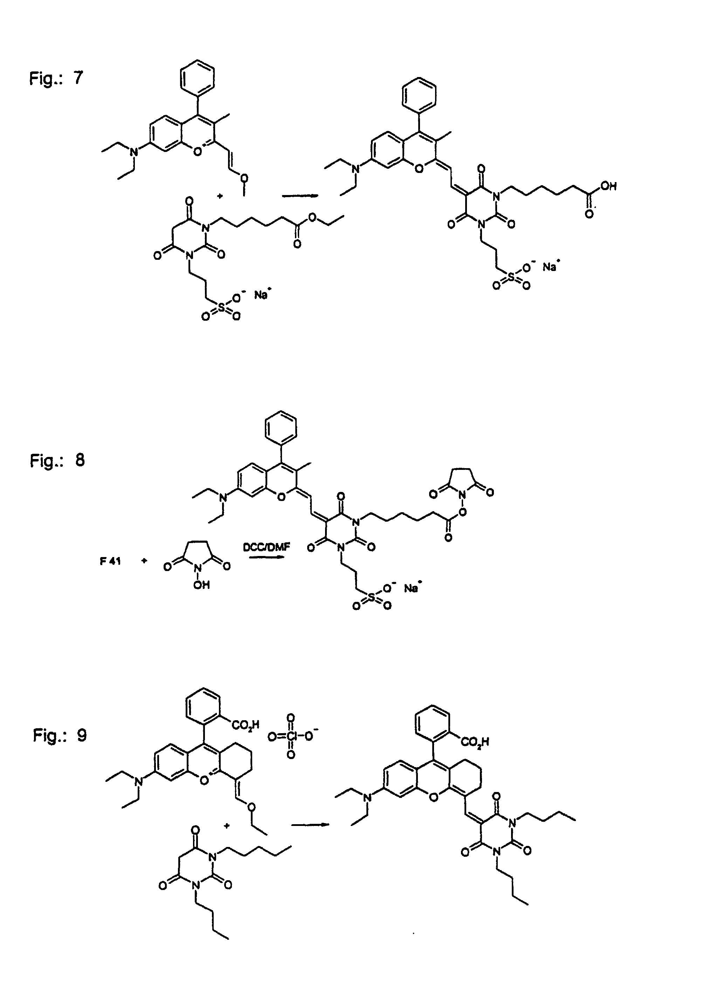

### 4. Natriumsalz des 3-[5-{2-[4-(Phenyl)-7-(diethylamino)-2H-2-chromenyliden]ethyliden}-3-(5-carboxypentyl)-2,4,6-trioxohexahydro-1-pyrimidinyl]-1-propansulfonats (F 40), vgl. Fig. 6:

253 mg (0,60 mmol) 4-(Phenyl)-7-(diethylamino)-2-[(*E*)-2-methoxy-1-ethenyl]chromenium-tetrafluoroborat und 249 mg (0,60 mmol) Natriumsalz des 1-*N*-(5'-Ethoxycarbonyl)-pentyl-3-*N'*-3'-sulfonatopropyl-2,4,6-trioxo-5H-pyrimidins werden nach Ausführungsbeispiel 1, Variante A zur Reaktion gebracht und aufgearbeitet. 162 mg (39 %) Ausbeute. ― UV/Vis (Ethanol): λₘₐₓ (ε) = 629 nm (78.400 |· mol⁻¹· cm⁻¹). ― Fluoreszenz (Ethanol): λₑₘ = 670 nm. ― MS (ESI^{⊖}): 664 (M^{⊖}), 332 (M-H)^{2⊖}. ― C₃₄H₃₈NaN₃O₉S (687,73)

### 5. Natriumsalz des 3-[5-{2-[3-(Methyl)-4-(phenyl)-7-(diethylamino)-2H-2-chromenyliden]-ethyliden}-3-(5-carboxypentyl)-2,4,6-trioxohexahydro-1-pyrimidinyl]-1-propansulfonats (F 41), vgl. Fig. 7:

261 mg (0,60 mmol) 3-(Methyl)-4-(phenyl)-7-(diethylamino)-2-[(*E*)-2-methoxy-1-ethenyl]-chromenium-tetrafluoroborat und 249 mg (0,60 mmol) Natriumsalz des 1-*N*-(5'-Ethoxy-carbonyl)-pentyl-3-*N'*-3'-sulfonatopropyl-2,4,6-trioxo-5H-pyrimidins werden nach Ausführungsbeispiel 1, Variante A zur Reaktion gebracht und aufgearbeitet. 185 mg (44 %) Ausbeute. ― UV/Vis (Ethanol): λₘₐₓ (ε) = 620 nm (64.900 |· mol⁻¹· cm⁻¹). ― Fluoreszenz (Ethanol): λₑₘ = 652 nm. ― MS (ESI^{⊖}): 678 (M^{⊖}), 339 (M-H)^{2⊖}. ― C₃₅H₄₀NaN₃O₉S (701,76).

### 6. Darstellung des NHS-Esters von F 41 mit N-Hydroxysuccinimid (NHS)/N,N'-Dicyclohexylcarbodiimid (DCC), vgl. Fig. 8:

15 mg **F 41,** 14 mg DCC und 4 mg NHS werden in 1 ml trockenem DMF gelöst. Dann gibt man 10 µl Triethylamin zu. Das Reaktionsgemisch wird 24 Stunden bei Raumtemperatur gerührt und anschließend filtriert. Im Anschluß daran wird das Lösungsmittel abgezogen und der Rückstand mit Ether gewaschen. Diese Reaktion verläuft quantitativ.

### 7. 1,3-Dibutyl-5-{[9-(2-carboxyphenyl)-6-(diethylamino)-2,3-dihydro-1H-4-xanthenyl]-methylen}-hexahydro-2,4,6-pyrimidintrion (F 33), vgl. Fig. 9:

158 mg (0,66 mmol) 1-*N*,3-*N*'-Dibutyl-2,4,6-trioxo-5H-pyrimidin und 351 mg (0,66 mmol) 9-(2-carboxyphenyl)-6-(diethylamino)-4-[(*E*)-1-ethoxymethyliden]-1 *H*,2*H*,3*H*,4*H*-xanthenium-perchlorat werden in 5 ml Acetanhydrid gelöst, mit ca. 1 ml Pyridin versetzt und bei RT für 2h belassen. Anschließend wird das Lösungsmittel im Vakuum abgezogen und der Rückstand durch Säulenchromatographie gereinigt (SiO₂, Eluent Toluol/Ethanol im Verhältnis 7:1).
194 mg (47 %) Ausbeute. ― UV/Vis (Ethanol): λₘₐₓ (ε) = 650 nm (80.000 |· mol⁻¹ · cm⁻¹). ― Fluoreszenz (Ethanol): λₑₘ = 670 nm. ― MS (ESI^{⊕}): 626 (M+H)^{⊕}. ― C₃₇H₄₃N₃O₆ (625,76).

### Herstellung der Vorprodukte C und D

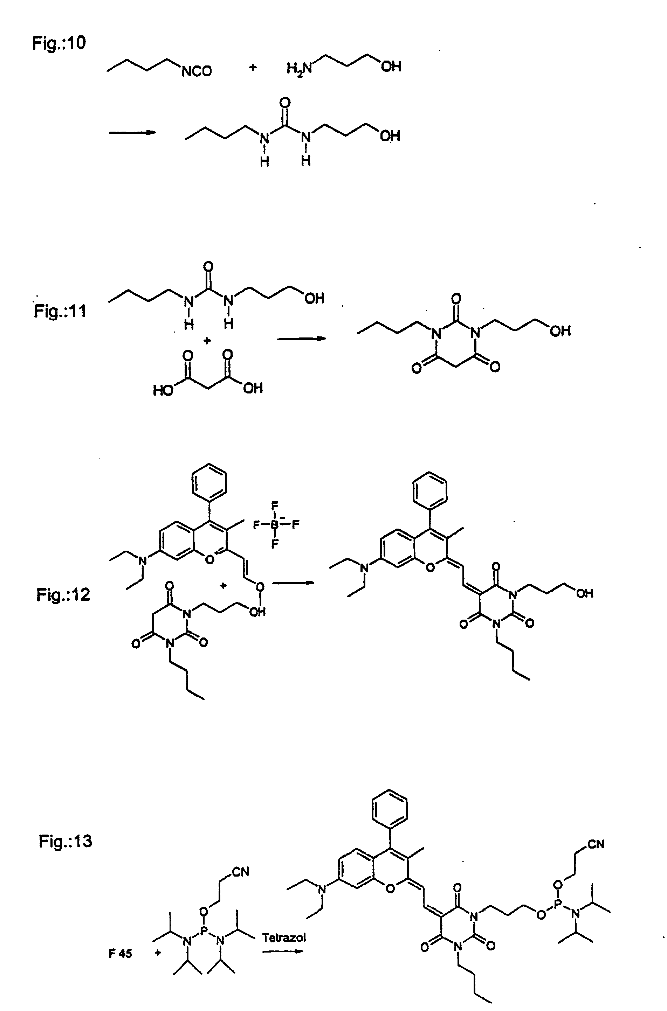

### C. N-(3-Hydroxypropyl)-N'-butyl-harnstoff, vgl. Fig. 10:

751 mg (10,0 mmol) 3-Aminopropanol werden in 5 ml Toluol gelöst und tropfenweise mit 991 mg (10,0 mmol) Butylisocyanat versetzt, wobei die Temperatur auf etwa 40 °C ansteigt. Beim Abkühlen fällt ein farbloser Niederschlag aus. Die Suspension wird auf 70 °C erwärmt, bis der Niederschlag wieder in Lösung geht. Der beim Abkühlen erneut ausfallende, voluminöse Niederschlag wird in etwa 3 ml Toluol aufgenommen, abgesaugt, mit Toluol und Ether gewaschen und im Vakuum getrocknet. 1,57 g (90 %) Ausbeute. ― ¹H NMR (250 MHz, CDCl₃): 0.92 (t, 3H), 1.36 (m, 2H), 1.46 (m, 2H), 1.65 (m, 2H), 3.14 (dt, 2H), 3.32 (dt, 2H), 3.65 (dt, 2H), 4.21 (t, 1 H), 5.22 (t, 1H), 5.44 (t, 1H). ― MS (ESI^{⊕}): 175 (M+H)^{⊕}. ― C₈H₁₈N₂O₂ (174,24).

### D. 1-N-(3'-Hydroxypropyl)-3-N'-butyl-2,4,6-trioxo-5H-pyrimidin, vgl. Fig. 11:

1011 mg (5,8 mmol) *N*-3-Hydroxypropyl-*N*'-butyl-harnstoff und 624 mg (6,0 mmol) Malonsäure werden in 4 ml Essigsäure gelöst und auf 60 °C erwärmt. Zu dieser Lösung tropft man langsam 8 ml Acetanhydrid und hält die Lösung für 6 Stunden auf 100 °C. Nach dem Abkühlen auf Raumtemperatur wird das Lösungsmittel im Vakuum abgezogen. Der verbleibende ölige Rückstand wird im Hochvakuum getrocknet und ohne weitere Reinigung zur Farbstoffsynthese eingesetzt. 1,42 g (86 %) Rohausbeute.

### Ausführungsbeispiele 8 - 17 (Verbindungen der allgemeinen Formel II)

**8.** 1-Butyl-5-{2-[3-(methyl)-4-(phenyl)-7-(diethylamino)-2*H*-2-chromenyliden] ethyliden}-3-(3-hydroxypropyl)hexahydro-2,4,6-pyrimidintrion (**F 45**), vgl. Fig. 12: 261 mg (0,60 mmol) 3-(Methyl)-4-(phenyl)-7-(diethylamino)-2-[(*E*)-2-methoxy-1-ethenyl]-chromenium-tetrafluoroborat und 171 mg (0,60 mmol) 1-*N*-(3'-Hydroxypropyl)-3-*N'*-butyl-2,4,6-trioxo-5H-pyrimidin werden in 20 ml Ethanol gelöst, mit ca. 1 ml Triethylamin versetzt und kurz auf etwa 70 °C erwärmt. Nach dem Abkühlen auf Raumtemperatur beläßt man noch für 30 Minuten, zieht dann das Lösungsmittel im Vakuum ab und chromatographiert den Rückstand (SiO₂, Eluent Toluol/Ethanol im Verhältnis 7:3).
110 mg (33 %) Ausbeute. ― UV/Vis (Ethanol): λₘₐₓ (ε) = 622 nm (92.000 |· mol⁻¹· cm⁻¹). ― Fluoreszenz (Ethanol): λₑₘ = 654 nm. ― ¹H NMR (400 MHz, CDCl₃): einige Signale treten doppelt auf (Regioisomerie bezüglich Hydroxypropylgruppe): 0.93/0.95 (t, 3H), 1.18/1.20 (t, 6H), 1.38 (m, 2H), 1.62 (m, 2H), 1.88 (m, 2H), 1.96/1.98 (s, 3H), 3.42/3.43 (q, 4H), 3.54 (m, 2H), 3.95 (m, 2H), 4.14 (m, 2H), 6.50 (d, 1H), 6.67 (d, 1 H), 6.80 (d, 1 H), 7.18 (d, 2H), 7.47 (m, 3H), 7.64/7.66 (d, 1 H), 8.96/8.98 (d, 1H). ― MS (ESI^{⊕}): 558 (M+H)^{⊕}. ― C₃₃H₃₉N₃O₅ (557,69).

### 9. Darstellung des Phosphoramidits von F 45 mit 2-Cyanoethyl-bis-N,N,N,'N'tetraisopropyl-phosphoramidit, vgl. Fig. 13:

50 mg (0,09 mmol) **F 45** und 36 mg (0,12 mmol) 2-Cyanoethyl-bis-*N,N,N',N'*tetraisopropyl-phosphoramidit werden in trockenem Dichlormethan gelöst. Die Kondensationsreaktion wird durch Zugabe von 1,4 mg (0,02 mmol) Tetrazol gestartet und im Anschluß daran 2 Stunden gerührt. Nach Beendigung der Reaktion wird das Lösungsmittel im Vakuum abgezogen, der verbleibende Feststoff 3 mal mit je 15 ml Diethylether gewaschen und im Hochvakuum getrocknet. Diese Reaktion verläuft quantitativ.

### 10. 1,3-Dibutyl-5-{2-[2-(tert-butyl)-7-(diethylamino)-4H-4-chromenyliden]ethyliden}hexa-hydro-2,4,6-pyrimidintrion (F 34), vgl. Fig. 14:

241 mg (0,60 mmol) 2-(*tert*-Butyl)-7-(diethylamino)-4-[(*E*)-2-methoxy-1-ethenyl]-chromenium-tetrafluoroborat und 144 mg (0,60 mmol) 1-*N*,3-*N'*-Dibutyl-2,4,6-trioxo-5*H*-pyrimidin werden in 5 ml Acetanhydrid gelöst, mit ca. 1 ml Pyridin versetzt und bei RT für 2h belassen. Anschließend wird das Lösungsmittel im Vakuum abgezogen und der Rückstand durch Säulenchromatographie gereinigt (SiO₂, Eluent Toluol/Ethanol im Verhältnis 7:1).
175 mg (56 %) Ausbeute. ― UV/Vis (Ethanol): λₘₐₓ (ε) = 557 nm (152.600 |· mol⁻¹· cm⁻¹).- Fluoreszenz (Ethanol): λₑₘ = 576 nm. ― ¹H NMR (250 MHz, CDCl₃): 0.92 (t, 3H), 0.93 (t, 3H), 1.22 (t, 6H), 1.34 (s, 9H), 1.35 (m, 4H), 1.62 (m, 4H), 3.42 (q, 4H), 3.94 (t, 4H), 6.40 (s, 1H), 6.70 (dd, 1 H), 6.93 (s, 1 H), 8.02 (d, 1 H), 8.29 (d, 1 H), 8.67 (d, 1H). ― ¹³C NMR (62 MHz, CDCl₃): 12.53, 13.83, 13.85, 20.30, 20.35, 28.13, 30.41, 30.48, 36.59, 40.84, 41.47, 44.84, 96.53, 98.80, 102.89, 107.46, 110.57, 111.68, 126.94, 148.96, 151.83, 151.89, 152.05, 156.61, 163.10, 164.02, 169.59. ― MS (ESI^{⊕}): 522 (M+H)^{⊕}, 1043 (2M+H)^{⊕}. ― C₃₁H₄₃N₃O₄ (521,70).

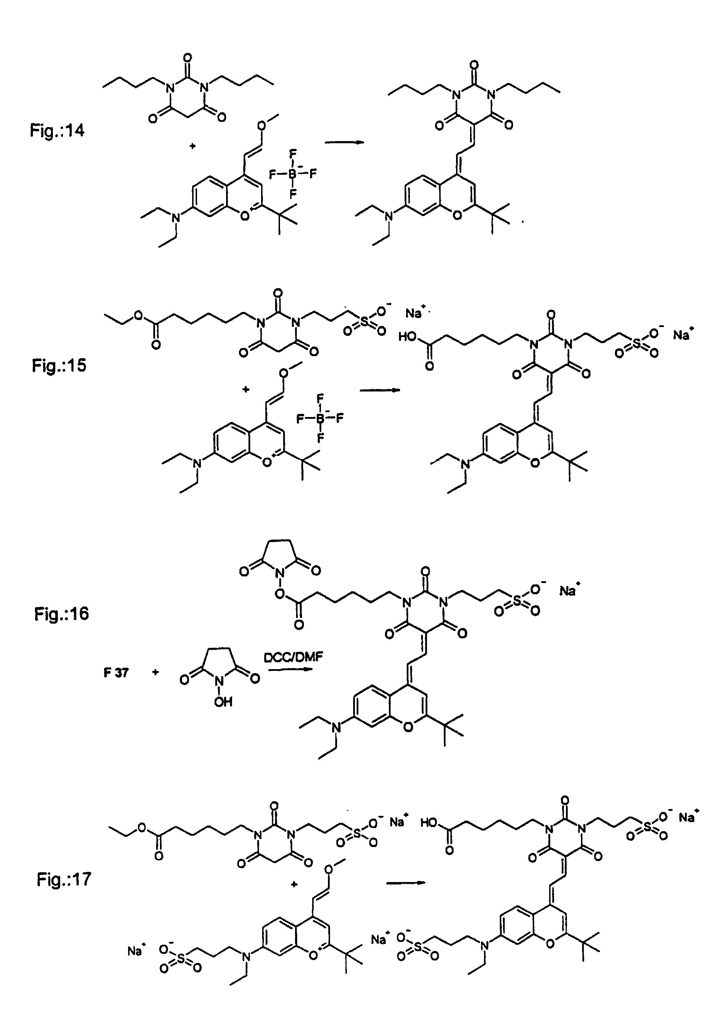

### 11. Natriumsalz des 3-[5-{2-[2-(tert-Butyl)-7-(diethylamino)-4H-4-chromenyliden]ethyliden}- 3-(5-carboxypentyl)-2,4,6-trioxohexahydro-1-pyrimidinyl]-1-propansulfonats (F 37/DY-550), vgl. Fig. 15:

### Variante A

241 mg (0,60 mmol) 2-(*tert*-Butyl)-7-(diethylamino)-4-[(*E*)-2-methoxy-1-ethenyl]-chromen-ium-tetrafluoroborat und 249 mg (0,60 mmol) Natriumsalz des 1-*N*-(5'-Ethoxycarbonyl)-pentyl-3-*N'*-3'-sulfonatopropyl-2,4,6-trioxo-5*H*-pyrimidins werden in 20 ml Ethanol gelöst, mit ca. 1 ml Triethylamin versetzt und kurz auf etwa 70 °C erwärmt. Nach dem Abkühlen auf Raumtemperatur beläßt man noch für 30 Minuten, zieht dann das Lösungsmittel im Vakuum ab und chromatographiert den Rückstand (SiO₂, Eluent Toluol/Ethanol im Verhältnis 7:3).
Der isolierte Ester wird in einer Mischung aus 10 ml Aceton und 10 ml 2-molarer Salzsäure 2 Stunden unter Rückfluß gekocht, die abgekühlte Reaktionslösung mit NaHCO₃ neutralisiert und das Lösungsmittel im Vakuum abgezogen. Der in Methanol lösliche Rückstand wird erneut chromatographiert (SiO₂ RP 18, Eluent Methanol/Wasser im Verhältnis 6:4).
182 mg (45 %) Ausbeute. ― UV/Vis (Ethanol): λₘₐₓ (ε) = 557 nm (122.300 l·mol⁻¹·cm⁻¹). - Fluoreszenz (Ethanol): λₑₘ = 578 nm. ― MS (ESI^{⊖}): 644 (M^{⊖}), 322 (M-H)^{2⊖}. ― C₃₂H₄₂NaN₃O₉S (667,74).

### Variante B

216 mg (0,60 mmol) 2-(*tert*-Butyl)-7-(diethylamino)-4-methylchromeniumtetrafluoroborat und 274 mg (0,60 mmol) Natriumsalz des 1-*N*-(5'-Ethoxycarbonyl)-pentyl-5-(methoxy-methylen)-3-*N'*-3'-sulfonatopropyl-2,4,6-trioxohexahydro-1-pyrimidins werden in 20 ml Ethanol gelöst, mit ca. 1 ml Triethylamin versetzt und kurz auf etwa 70 °C erwärmt. Die weitere Aufarbeitung der Reaktionslösung erfolgt nach Variante A.

### Variante C

216 mg (0,60 mmol) 2-(*tert*-Butyl)-7-(diethylamino)-4-methylchromeniumtetrafluoroborat, 249 mg (0,60 mmol) Natriumsalz des 1-*N*-(5'-Ethoxycarbonyl)-pentyl-3-*N*'-3'-sulfonato-propyl-2,4,6-trioxo-5H-pyrimidins, 1 ml Trimethoxymethan und 1 ml Pyridin werden in 10 ml Acetanhydrid gelöst und 1 Stunde unter Rückfluß erhitzt. Die weitere Aufarbeitung der Reaktionslösung erfolgt nach Variante A.

### 12. Darstellung des NHS-Esters von F 37 mit N-Hydroxysuccinimid (NHS)/N,N'-Dicyclo-hexylcarbodiimid (DCC), vgl. Fig. 16:

15 mg **F 37,** 14 mg DCC und 4 mg NHS werden in 1 ml trockenem DMF gelöst. Dazu gibt man 10 µl Triethylamin. Im Anschluß daran wird das Reaktionsgemisch 24 Stunden bei Raumtemperatur gerührt und daraufhin filtriert. Danach wird das Lösungsmittel abgezogen und der Rückstand mit Ether gewaschen. Diese Reaktion . verläuft quantitativ.

### 13. Dinatriumsalz des 3-[5-(2-{2-tert-Butyl-7-[ethyl-(3-sulfonatopropyl)-amino]-4H-4-chromenyliden}-ethyliden)-3-(5-carboxypentyl)-2,4,6-trioxohexahydro-1-pyrimidinyl]-1-propansulfonats (V03-01173), vgl. Fig. 17

219 mg (0,60 mmol) 2-*tert*-Butyl-7-[ethyl-3-sulfonatopropyl)-amino]-4-methylchromenium (Betain) und 274 mg (0,60 mmol) Natriumsalz des 1-*N*-(5'-Ethoxycarbonyl)pentyl-5-(methoxymethylen)-3-*N'*-3'-sulfonatopropyl-2,4,6-trioxohexahydro-1-pyrimidins werden in 20 ml Methanol gelöst, mit ca. 1 ml Triethylamin versetzt und kurz zum Sieden erhitzt. Die weitere Aufarbeitung der Reaktionslösung erfolgt nach Ausführungsbeispiel 11, Variante A. 165 mg (35 %) Ausbeute. ― UV/Vis (Ethanol): λₘₐₓ (ε) = 556 nm (142.000 |· mol⁻¹· cm⁻¹). ― Fluoreszenz (Ethanol): λₑₘ = 580 nm. ― MS (ESI^{⊖}): 369 (M)^{2⊖}. ― C₃₃H₄₃Na₂N₃O₁₂S₂ (783,80).

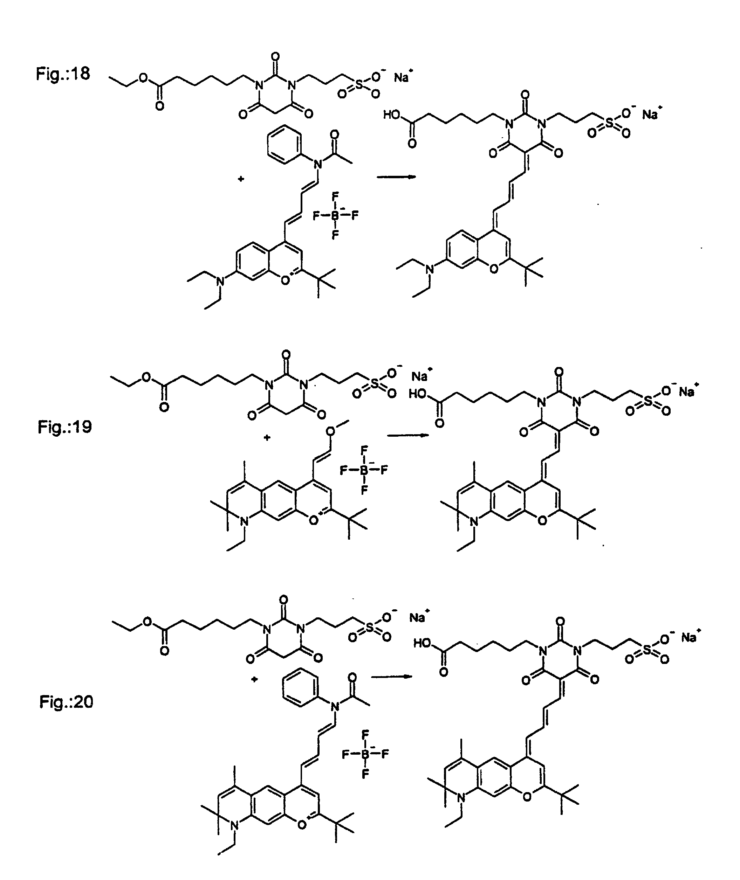

### 14. Natriumsalz des 3-[5-{(E)-4-[2-(tert-Butyl)-7-(diethylamino)-4H-4-chromenyliden]-2-butenyliden}-3-(5-carboxypentyl)-2,4,6-trioxohexahydro-1-pyrimidinyl]-1-propansulfonats (F 38), vgl. Fig. 18:

318 mg (0,60 mmol) *N*-1-{(1*E*,3*E*)-4-[2-(*tert*-Butyl)-7-(diethylamino)-4-chromeniumyl]-1,3-butadienyl}-*N*-1-phenylacetamid-tetrafluoroborat werden mit 249 mg (0,60 mmol) ―Natriumsalz des 1-*N*-5'-(Ethoxycarbonyl)-pentyl-3-*N'*-3'-sulfonatopropyl-2,4,6-trioxo-5H-pyrimidins nach Ausführungsbeispiel 11, Variante A zur Reaktion gebracht und aufgearbeitet.
148 mg (36 %) Ausbeute. ― UV/Vis (Ethanol): λₘₐₓ (ε) = 655 nm (145.000 l· mol⁻¹·cm⁻¹). ― Fluoreszenz (Ethanol): λₑₘ = 678 nm. ― ¹H NMR (250 MHz, D₂O): 0.97 (t, 6H), 1.15 (s, 9H), 1.23 (m, 2H), 1.48 (m, 4H), 1.89 (m, 2H), 2.12 (m, 2H), 2.83 (t, 2H), 3.15 (s, br, 4H), 3.59 (s, br, 2H), 3.74 (s, br, 2H), 5.93 (d, 1H), 6.06 (s, 1H), 6.15 (s, 1H), 6.55 (d, 1 H), 6.81 (m, 1 H), 6.95 (m, 2H), 7.19 (d, 1H). ― MS (ESI^{⊖}): 670 (M^{⊖}), 335 (M-H)^{2⊖}· ― C₃₄H₄₄NaN₃O₉S (693,78).

### 15. Natriumsalz des 3-[5-{2-[2-(tert-Butyl)-9-ethyl-6,8,8-trimethyl-8,9-dihydro-4Hpyrano-[3,2-g]chinolin-4-yliden]ethyliden}-3-(5-carboxypentyl)-2,4,6-trioxohexahydro-1-pyrimidinyl]-1-propansulfonats (F 48), vgl. Fig. 19:

272 mg (0,60 mmol) 2-(*tert*-Butyl)-9-ethyl-4-[(*E*)-2-methoxy-1-ethenyl]-6,8,8-trimethyl-8*H*,9*H*-pyrano[3,2-*g*]chinolin-1-ium-tetrafluoroborat werden mit 249 mg (0,60 mmol) Natriumsalz des 1-*N*-(5'-Ethoxycarbonyl)-pentyl-3-*N*'-3'-sulfonatopropyl-2,4,6-trioxo-5H-pyrimidins nach Ausführungsbeispiel 11, Variante A zur Reaktion gebracht und aufgearbeitet.
180 mg (42 %) Ausbeute. ― UVNis (Ethanol): λₘₐₓ (ε) = 574 nm (132.000 |· mol⁻¹· cm⁻¹). ― Fluoreszenz (Ethanol): λₑₘ = 605 nm. ― MS (ESI^{⊖}): 696 (M^{⊖}), 348 (M-H)^{2⊖}. ― C₃₆H₄₆NaN₃O₉S (719,82).

### 16. Natriumsalz des 3-[5-{4-[2-(tert-Butyl)-9-ethyl-6,8,8-trimethyl-8,9-dihydro-4Hpyrano-[3,2-g]chinolin-4-yliden]-2-butenyliden}-3-(5-carboxypentyl)-2,4,6-trioxohexahydro-1-pyrimidinyl]-1-propansulfonats (F 50), vgl. Fig. 20:

350 mg (0,60 mmol) *N*-1-{(1*E*,3*E*)-4-[2-(*tert*-Butyl)-9-ethyl-6,8,8-trimethyl-8*H*,9*H*pyrano[3,2*g*]chinolin-1-ium-4-yl]-1,3-butadienyl}-*N*-1-phenylacetamid-tetrafluoroborat werden mit 249 mg (0,60 mmol) Natriumsalz des 1-*N*-(5'-Ethoxycarbonyl)-pentyl-3-*N*'-3'-sulfonatopropyl-2,4,6-trioxo-5H-pyrimidins nach Ausführungsbeispiel 11, Variante A zur Reaktion gebracht und aufgearbeitet. 144 mg (32 %) Ausbeute. ― UV/Vis (Ethanol): λₘₐₓ (ε) = 671 nm (175.000 |· mol⁻¹·cm⁻¹). - Fluoreszenz (Ethanol): λₑₘ = 700 nm. ― MS (ESI^{⊖}): 722 (M^{⊖}), 361 (M-H)^{2⊖}. ― C₃₈H₄₈NaN₃O₉S (745,86).

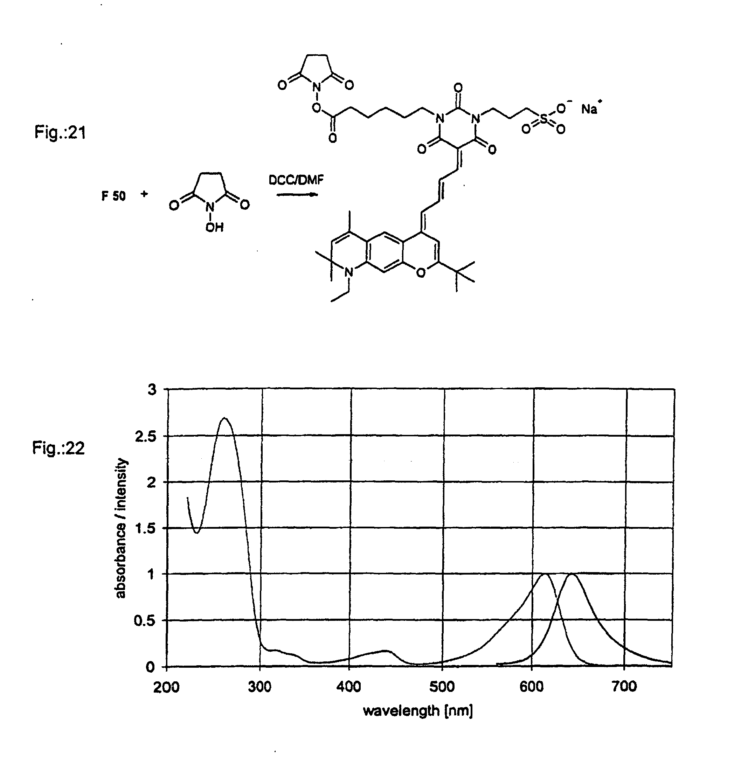

### 17. Darstellung des NHS-Esters von F 50 mit N-Hydroxysuccinimid (NHS)/N,N'-Dicyclo-hexylcarbodiimid (DCC), vgl. Fig. 21:

15 mg **F 50,** 14 mg DCC und 4 mg NHS werden in 1 ml trockenem *N*,*N*-Dimethylformamid (DMF) gelöst. Dazu gibt man 10 µl Triethylamin. In Anschluß daran wird das Reaktionsgemisch 24 Stunden bei Raumtemperatur gerührt und anschließend filtriert. Danach wird das Lösungsmittel abgezogen und der Rückstand mit Ether gewaschen. Diese Reaktion verläuft quantitativ.

### 18. Absorptions- und Emissionsspektrum eines DY-610-DNA-Konjugats, vgl. Fig. 22:

Der **F-39-NHS-Ester** (Ausführungsbeispiel 2, Figur 4) (0,2 mg in 200 µl DMF) wird in Boratpuffer (0.1 M, pH 8.5) mit einem 5'-aminomodifizierten DNA-Oligomer (23mer) unter Schütteln bei 37 °C über Nacht umgesetzt. Im Anschluß daran wird das Reaktionsgemisch mit 2,1 ml destilliertem Wasser versetzt. Das gesamte Volumen wird nun auf eine NAP25-Säule (*Amersham Pharmacia*) gegeben und mit 3,5 ml Wasser eluiert. Das gesammelte Eluat wird dann im Vakuum getrocknet. Die HPLC-Reinigung des Konjugats erfolgt auf einer RESOURCE™ RPC-Säule (*Amersham Pharmacia*) mit Triethylammoniumacetat (100 mM, pH 6,9) und Triethylammoniumacetat (100 mM, pH 6,9) / Acetonitril (1:1) als Eluenten.
Das Absorptionsspektrum wurde auf einem *Lambda 16*-Spektrophotometer (*Perkin-Elmer*) in destilliertem Wasser aufgenommen. Das Fluoreszenzspektrum wurde auf einem *Aminco-Bowman* 2-Fluorimeter (Anregungswellenlänge 550 nm, Spalt 4/4) registriert. Beide Spektren, normiert auf das langwelligste Absorptions- bzw. das Emissionsmaximum, sind in Fig. 22 dargestellt.

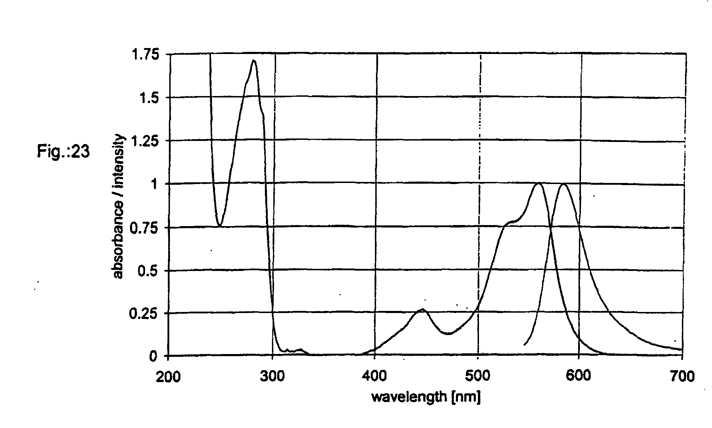

### 19. Markieren von Avidin mit DY-550-NHS-Ester, vgl. Fig. 23:

0,49 mg (641 nmol) DY-550-NHS-Ester (Ausführungsbeispiel 12, Figur 16) werden in 106 µl N,N-Dimethylformamid (DMF) gelöst. Ebenfalls wird 1 mg Avidin (15,15 nmol) in 200 µl Bicarbonatpuffer (pH 9,0, 50 mM) gelöst. Dann werden 5 µl der DY-550-NHS-Ester-Lösung zu der Avidin-Lösung pipettiert. Nun wird dieser Markierungsansatz eine Stunde bei Raumtemperatur im Dunkeln unter gelegentlichem Schütteln belassen. Danach wird das entstandene DY-550-Avidin-Konjugat auf einer Sephadex-Säule (Sephadex G 25 medium) von nicht reagiertem bzw. hydrolysiertem NHS-Ester abgetrennt, wobei Phosphatpuffer (pH 7,2, 22 mM, 0,1% Azid) als Laufmittel verwendet wird. Die so gereinigte DY-550-Avidin-Konjugat-Lösung wird auf 1 ml aufgefüllt (ebenfalls mit Phosphatpuffer, pH 7,2, 22 mM) und durch die Aufnahme eines Absorptions- und Fluoreszenzspektrums charakterisiert. Dazu wird die Konjugat-Lösung auf ein Zehntel mit dem bereits genannten Phosphatpuffer verdünnt und in einer 1 cm-Quarzküvette (vierseitig offen) am Absorptionsspektrometer *Lambda 16* (*Perkin-Elmer*) und dem Fluoreszenzspektrometer *Aminco-Bowman* 2 (Spalt 4/4, PMT-Spannung 700 V) vermessen. Figur 23 zeigt die erhaltenen, auf das Absorptions- bzw. Emissionsmaximum des Farbstoffs normierten Spektren.

## Patentansprüche

1. Marker-Farbstoffe auf der Basis von Merocyaninen der allgemeinen Formeln **I** und/oder **II,** 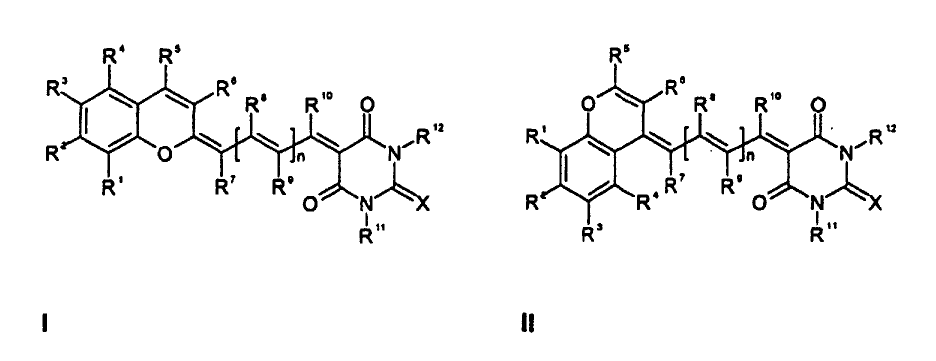
wobei R¹ bis R¹² gleich oder unterschiedlich sind und jeweils Wasserstoff, Chlor, Brom, einen aliphatischen oder einkemigen aromatischen Rest, jeweils mit insgesamt höchstens 12 Kohlenstoffatomen bedeuten, die als substituierter Rest außer Kohlenstoff und Wasserstoff noch bis zu 4 Sauerstoffatome und entweder 0, 1 oder 2 Stickstoffatome oder ein Schwefelatom oder ein Schwefel- und ein Stickstoffatom enthalten können oder eine Aminfunktion sind, an deren Stickstoffatom Wasserstoff oder Substituenten mit zusammen höchstens 8 Kohlenstoffatomen gebunden sind, wobei diese Substituenten aus Kohlenstoff, Wasserstoff und höchstens zwei Sulfonsäuregruppen bestehen, wobei R¹ und R², R² und R³, R³ und R⁴, R⁵ und R⁶ und R⁶ und R⁷ jeweils zusammen einen aromatischen oder nichtaromatischen Kohlenwasserstoff- oder stickstoffhaltigen 5-oder 6-Ring bilden können, mit der Maßgabe, dass R² eine Aminfunktion darstellt und dass wenigstens einer der Reste R¹ und R³ für sich einen SO₃⁻ -Rest darstellen kann,
X Sauerstoff oder Schwefel bedeutet und
n = 0 oder eine ganze Zahl von 1 - 3 bedeutet, wobei die für n = 2 oder 3 doppelt oder dreifach vorkommenden Substituenten R⁸ und R⁹ gleich oder verschieden sein können.

2. Verbindungen nach Anspruch 1, **dadurch gekennzeichnet, dass** wenigstens einer der Reste R¹ bis R¹² eine solubilisierende oder ionisierbare Gruppe enthält, die vorzugsweise über eine aliphatische oder heteroaliphatische Gruppe an das Grundgerüst gebunden ist.

3. Verbindungen nach Anspruch 2, **dadurch gekennzeichnet, dass** die solubilisierende bzw. ionisierbare Gruppe SO₃⁻, CO₂H oder OH oder eine Kombination davon ist.

4. Verbindungen nach einem oder mehreren der Ansprüche 1 - 3, **dadurch gekennzeichnet, dass** wenigstens einer der Substuenten R¹ bis R¹² eine reaktive Gruppe enthält, die zur Reaktion mit Biomolekülen unter Ausbildung einer kovalenten Bindung fähig ist.

5. Verbindungen nach Anspruch 4, **dadurch gekennzeichnet, dass** die reaktive Gruppe eine *N*-Hydroxysuccinimidester-, Maleimid- oder eine Phosphoramidit-Gruppe ist.

6. Verbindungen nach Anspruch 4 oder 5, **dadurch gekennzeichnet, dass** aliphatische Reste R¹ bis R¹² 1 - 6 Kohlenstoffatome enthalten, die in ω-Stellung durch eine SO₃⁻-, SO₂Cl-, HO-, oder Carboxyl-Gruppe substituiert sind, die ihrerseits zu Estern umgesetzt sein können.

7. Verbindungen nach einem oder mehreren der vorhergehenden Ansprüche, **dadurch gekennzeichnet, dass** R², R¹¹ und R¹² frei von Chlor und Brom sind, sonst in höchstens 4, zweckmäßig in höchstens 2 und vorzugsweise in keinem der Reste R¹ und R³ bis R¹⁰ Chlor und/oder Brom enthalten ist.

8. Verbindungen nach einem oder mehrerer der vorhergehenden Ansprüche, **dadurch gekennzeichnet, dass** die Reste in den Substituenten R¹ bis R¹² jeweils höchstens 10, vorzugsweise höchstens 7 Kohlenstoffatome und 0 bis 2 SO₃⁻-Gruppen enthalten.

9. Verbindungen nach einem oder mehreren der vorhergehenden Ansprüche, **dadurch gekennzeichnet, dass** die Reste R¹ bis R¹² außer der Aminfunktion von R² insgesamt höchstens 2 Stickstoffatome enthalten.

10. Verbindungen nach einem oder mehreren der vorhergehenden Ansprüche, **dadurch gekennzeichnet** durch wenigstens eines der Merkmale, dass a) X = O und b) n = 0, 1 oder 2 bedeuten und c) aliphatische Reste als Kohlenwasserstoffreste 1 - 4 Kohlenstoffatome enthalten.

11. Verwendung der Verbindungen gemäß einem oder mehreren der Ansprüche 1 - 10 als Farbstoffkomponente zur optischen Markierung biologischer und organischer Substanzen.

## Claims

1. Marker dyes based on merocyanines having the general formulas I and/or II, 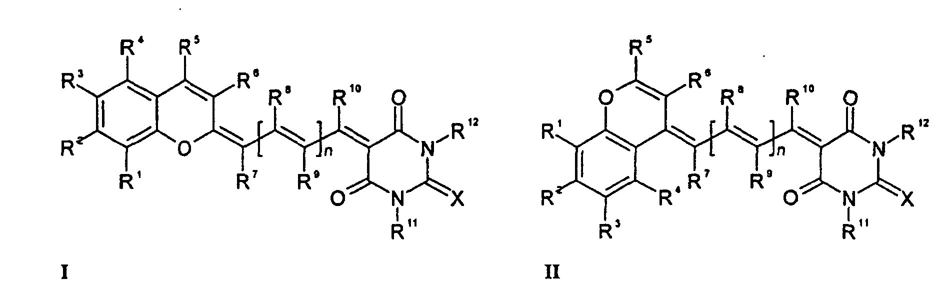
wherein R¹ - R¹² are the same or different and can represent any of the following: hydrogen, chlorine, bromine, or an aliphatic or mononuclear aromatic group with in each case a total of at most 12 carbon atoms; each may contain as a substituted residue not only carbon and hydrogen but also up to 4 oxygen atoms and either 0, 1 or 2 nitrogen atoms or a sulphur atom or both a sulphur and a nitrogen atom, or represent an amino function, to the nitrogen atom of which is bound hydrogen or substituents together comprising at most 8 carbon atoms, said substituents consisting of carbon, hydrogen and at most two sulphonic-acid groups, wherein each of the pairs R¹ and R², R² and R³, R³ and R⁴, R⁵ and R⁶ and R⁶ and R⁷ together can form an aromatic or non-aromatic hydrocarbon- or nitrogen-containing 5- or 6-membered ring, with the proviso that R² represents an amino function and that at least one of the residues R¹ and R³ *per se* may represent an SO₃⁻ residue,
X represents oxygen or sulphur and
n = 0 or an integer from 1 to 3, with the proviso that when n is 2 or 3 the substituents R⁸ and R⁹, which are present twice or three times, may be the same or different.

2. Compounds according to Claim 1, **characterized in that** at least one of the residues R¹ to R¹² contains a solubilizing or ionizable group, which preferably is bound to the skeleton by way of an aliphatic or heteroaliphatic group.

3. Compounds according to Claim 2, **characterized in that** the solubilizing or ionizable group is SO₃⁻, CO₂H or OH or a combination thereof.

4. Compounds according to one or more of the claims 1-3, **characterized in that** at least one of the substituents R¹ to R¹² contains a reactive group that is capable of reacting with biomolecules to form a covalent bond.

5. Compounds according to Claim 4, **characterized in that** the reactive group is an *N*-hydroxysuccinimide ester group, a maleimide group or a phosphoramidite group.

6. Compounds according to Claim 4 or 5, **characterized in that** aliphatic residues R¹ to R¹² contain from 1 to 6 carbon atoms, substituted in the ω position by a SO₃⁻, SO₂Cl, HO, or carboxyl group, which in turn can be converted to esters.

7. Compounds according to one or more of the preceding claims, **characterized in that** R², R¹¹ and R¹² are free from chlorine and bromine, and that of the remaining residues, namely R¹ and R³ to R¹⁰, at most 4, advantageously no more than 2, and preferably none contain chlorine and/or bromine.

8. Compounds according to one or more of the preceding claims, **characterized in that** the residues in the substituents R¹ to R¹² each contain at most 10, preferably no more than 7 carbon atoms and 0 to 2 SO₃⁻ groups.

9. Compounds according to one or more of the preceding claims, **characterized in that** the residues R¹ to R¹², apart from the amino function of R², altogether contain at most 2 nitrogen atoms.

10. Compounds according to one or more of the preceding claims, **characterized by** at least one of the following features: (a) X = zero and (b) n = 0, 1 or 2, and (c) aliphatic residues in the form of carbohydrate residues contain from 1 to 4 carbon atoms.

11. Application of the compounds according to one or more of the claims 1 to 10 as dye components for the optical labeling of biological and organic substances.

## Revendications

1. Colorants marqueurs à base de mérocyanines des formules générales I et/ou II 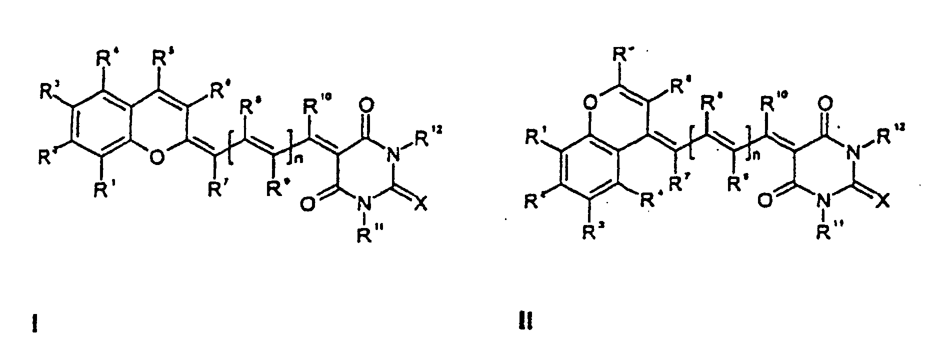
dans lesquelles R¹ à R¹² sont identiques ou différents et représentent chacun l'hydrogène, le chlore, le brome, un résidu aliphatique ou aromatique monocyclique, respectivement avec 12 atomes de carbone au maximum, qui, comme résidu substitué, peuvent contenir, outre le carbone et l'hydrogène, encore jusqu'à 4 atomes d'oxygène et soit 0, 1, ou 2 atomes d'azote ou un atome de soufre, soit un atome de soufre et un atome d'azote, ou sont une fonction amine sur l'atome d'azote de laquelle est ou sont lié(s) l'hydrogène ou des substituants avec 8 atomes de carbone au maximum, ces substituants étant constitués de carbone, d'hydrogène et de deux groupes d'acide sulfonique au maximum, R¹ et R², R² et R³, R³ et R⁴, R⁵ et R⁶ et R⁶ et R⁷ pouvant former ensemble un cycle à 5 ou 6 éléments aromatique ou non-aromatique hydrocarboné ou contenant de l'azote, étant précisé que R² représente une fonction amine et qu'au moins l'un des résidus R¹ et R³ peut représenter, quant à lui, un résidu de SO₃⁻,
X représente l'oxygène ou le soufre et
n = 0 ou représente un chiffre entier de 1 à 3, les substituants R⁸ et R⁹ qui se présentent en double ou en triple pour n = 2 ou 3 pouvant être identiques ou différents.

2. Composés selon la revendication 1, **caractérisés en ce qu'**au moins l'un des résidus R¹ à R¹² contient un groupe solubilisant ou ionisable qui est lié, de préférence, par un groupe aliphatique ou hétéroaliphatique, au squelette de base.

3. Composés selon la revendication 2, **caractérisés en ce que** le groupe solubilisant et/ou ionisable est SO₃⁻, CO₂H ou OH, ou une combinaison de ces éléments.

4. Composés selon l'une ou plusieurs des revendications 1 à 3, **caractérisés en ce qu'**au moins l'un des substituants R¹à R¹² contient un groupe réactif susceptible de réagir avec des biomolécules en formant une liaison covalente.

5. Composés selon la revendication 4, **caractérisés en ce que** le groupe réactif est un groupe d'esters de N-hydroxysuccinimide, un groupe maléimide ou un groupe phosphoramidite.

6. Composés selon la revendication 4 ou 5, **caractérisés en ce que** les résidus aliphatiques R¹ à R¹² contiennent 1 à 6 atomes de carbone qui, en position ω, sont substitués par un groupe SO₃⁻, SO₂Cl, HO ou carboxyle, qui, à son tour, peut être transformé en esters.

7. Composés selon l'une ou plusieurs des revendications précédentes, **caractérisés en ce que** R², R¹¹ et R¹² ne contiennent pas de chlore et de brome ; autrement, du chlore et/ou du brome est contenu dans tout au plus 4, avantageusement dans tout au plus 2 et de préférence dans aucun des résidus R¹ et R³ à R¹⁰.

8. Composés selon l'une ou plusieurs des revendications précédentes, **caractérisés en ce que** les résidus présents dans les substituants R¹ à R¹² contiennent chacun au maximum 10, de préférence au maximum 7 atomes de carbone et de 0 à 2 groupes SO₃⁻.

9. Composés selon l'une ou plusieurs des revendications précédentes, **caractérisés en ce que** les résidus R¹ à R¹² contiennent, hormis la fonction amine de R², tout au plus 2 atomes d'azote en tout.

10. Composés selon l'une ou plusieurs des revendications précédentes, **caractérisés par** au moins l'une des caractéristiques suivantes selon lesquelles : a) X = O et b) n = 0,1 ou 2 et c) les résidus aliphatiques, en tant que résidus d'hydrocarbures, contiennent de 1 à 4 atomes de carbone.

11. Utilisation des composés selon l'une ou plusieurs des revendications 1 à 10 comme composant colorant pour le marquage optique de substances biologiques et organiques.
